# FASCICULE DE BREVET EUROPEEN

(11) **EP 1 951 381 B1**
(45) Date de publication et mention de la délivrance du brevet: **22.10.2014**
(21) Numéro de dépôt: 06808202.3
(22) Date de dépôt: 26.09.2006
(51) Int. Cl.: C07C 233/12, C07C 233/36, C07C 233/62, C07C 233/78, C07D 213/30

(54) **NOUVEAUX POLYAMIDES PERFLUOROALKYLES LIPOPHOBES, LEUR OBTENTION ET LEUR UTILISATION**
NEUE LIPOPHOBE PERFLUORALKYLPOLYAMIDE, IHRE HERSTELLUNG UND IHRE VERWENDUNG
NOVEL LIPOPHOBIC PERFLUOROALKYL POLYAMIDES, PREPARATION THEREOF AND USE THEREOF

(30) Priorité: 23.11.2005 FR 0511823
(43) Date de publication de la demande: 06.08.2008
(73) Titulaire: SZONYI, Stéphane, 06000 Nice (FR); SZONYI, Stephane, 98000 Monaco (MC); SZONYI, François, 98000 Monaco (MC)
(72) Inventeur: SZONYI, Stéphane, 06000 Nice (FR); SZONYI, Stephane, 98000 Monaco (MC); SZONYI, François, 98000 Monaco (MC)
(86) Numéro de dépôt international: PCT/FR2006/002186
(87) Numéro de publication internationale: WO 2007/060300

(56) Documents cités:
- WO-A-90/03966
- FR-A1- 2 839 071
- FR-A1- 2 866 883

## Description

La présente invention concerne de nouveaux tensioactifs perfluoroalkylés de type amphotère dont le groupement perfluoré est issu de la télomérisation du tétrafluoroéthylène (TFE) et dont la structure moléculaire correspond à la formule générale suivante :

**A-B-(D-R_{F})ᵣ**

Ces composés ont la particularité de comporter, dans la partie **A,** un groupement acidoalkyl/arylamido lié à un groupement polyamide substitué

-**[NH-(CH₂)ₘ-N⁺(R₁)(R₂)-CH₂-CO]ₚ-,**

ou un groupement méthylène carboxylate lié à un groupement polyamide substitué

**-N⁺(R₁)(_{R2})-(CH₂)ₘ-NHCO-CH₂]ₚ-**

La partie A est séparée de la chaîne polyfluoroalkylée **R_{F}** par un groupement -**B-D**-.

Dans cette formule générale :
**R_{F}** est une chaîne perfluoroalkyle linéaire ou ramifiée **CₙF₂ₙ₊₁** où n = 4 à 20, ou un mélange de groupements perfluoroalkyle linéaires ou ramifiés ;
**A** est un groupement **X - E - CO -[NH - (CH₂)ₘ-N⁺(R₁)(R₂) - CH₂- CO]ₚ- NH -(CH₂)ₘ N⁺(R₁)(R₂)-**
ou **Na⁺⁻OOC-CH₂[-N⁺(R₁)(R₂)-(CH₂)ₘ₋NHCO-CH₂]p-N⁺(R₁)(R₂)-(CH₂)ₘ-NH-;**
**B** est un groupement **-CH₂CH(OH)CH₂-CH=CH-, -CH₂CH(OH)CH₂OCH₂CH(I)CH₂-, -CH₂CH(OH)CH₂OCH₂CH=CH₂-, -CH₂CH(OH)CH₂OC₂H₄-, -CH₂CH(OH)CH₂**-**SC₂H₄-, -CH₂CH(OH)CH₂(OC₂H₄)₁₋₃SC₂H₄-, -CH₂CH(OH)-, -CH₂CH(OH)CH₂-, -CH₂CH(OH)CH₂-O-C₃H₆SC₂H₄-, -CH₂CH(OH) CH₂-N=** ;
**D** est un groupement **-CH₂CH(I)CH₂-** et/ou **-CH₂CH=CH-** lorsque **B** est un groupement **-CH₂CH(OH) CH₂-N=**, néant dans les autres cas;
**E** est égal à néant ou à un groupement **-(CH₂)_{q}**-, 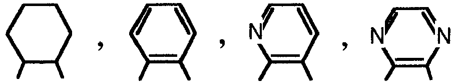
**M⁺ = un ion alcalin ou néant;**
**m, p** et **q** sont des nombres entiers valant 2 ou 3 indépendamment les uns des autres;
**r** est égal à **2** lorsque **B** est égal à **-CH₂CH(OH)CH₂-N=,** 1 dans les autres cas ;
**R₁** et **R₂** sont des groupements alkyle mineur identiques ;
**X** est un groupement alkyle ou aryle lorsque E est néant ou **-COO** lorsque **E** est différent de néant ;

De nombreux agents tensioactifs perfluoroalkylés de type carboxybétaïne de l'art antérieur ont été décrits dans les brevets FR 2.088.941, FR 2.185.668, DE 2.559.189, FR 2.390.426, US 4.069.244, US 4.126.633, US 4.283.533 , US 4.795.590.

Dans le brevet FR 2 839 071, les mêmes auteurs ont décrit de nouvelles polyamines ou polyamides perfluoroalkylées alcolipophobes de type amphotère, exemptes de groupement sulfone ou sulfonyle (SO₂), capables de réagir avec les amines, les polyamines, les polypeptides issus des protéines et les polysaccharides.

Les carboxybétaïnes perfluoroalkylées de cette invention sont caractérisées par leur grand pouvoir de réduction de la tension superficielle de l'eau et des solutions d'électrolytes inorganiques. En raison de l'abaissement important de la tension superficielle que les composés de l'invention produisent dans l'eau, ces derniers peuvent être utilisés comme agents mouillants, moussants, émulsifiants et dispersants.

Les composés de type carboxybétaïne sont particulièrement intéressants dans la mesure où ils peuvent être utilisés aussi bien dans les milieux fortement acides que basiques. Les agents tensioactifs perfluoroalkylés de l'invention sont particulièrement efficaces lorsqu'ils sont utilisés comme additifs pour les mousses extinctrices synthétiques (AFFF) et protéiniques (FFFP) filmogènes destinées à la lutte contre les incendies d'hydrocarbures ou de solvants. Ces mousses ont été décrites dans de nombreux brevets : US 4.042.522, US 4.090.967, DE 2.325.855, DE 2.732.555, DE 2.749.331, DE 2.829.594, EP 311.570, US 5.085.786, EP 780.141 (AFFF); US 3.475.333, DE 1.928.556, DE 2.240.263, FR 2.575.165 (FFFP).

Les avantages de cette invention sont multiples :
- les réactions permettant d'obtenir les tensioactifs perfluoroalkylés selon l'invention sont faciles et rapides à mettre en oeuvre,
- les composés selon l'invention sont aisément synthétisés à partir de produits disponibles dans le commerce par des procédés économiquement viables.,
- les composés selon l'invention sont préparés sans avoir à séparer et purifier chaque intermédiaire,
- les composés selon l'invention ne comportent aucun groupement perfluorooctane sulfonyle ou perfluorooctane carboxyle susceptible de se dégrader respectivement en perfluorooctane sulfonate (PFOS) ou perfluorooctane acide carboxylique (PFOA). Il faut rappeler que les tensioactifs fluorés possédant ce type de groupement dans leur structure moléculaire font actuellement l'objet d'études sur leur éventuelle Persistance, Bioaccumulation et Toxicité (PBT).

Mais l'une des originalités remarquable de cette invention réside dans le fait que les composés polyamidés selon l'invention, outre qu'ils permettent d'abaisser notablement la tension superficielle de l'eau, permettent de façon tout à fait inattendue d'obtenir un film aqueux à la surface des hydrocarbures, bien plus résistant que celui obtenu avec des tensioactifs provoquant un abaissement plus marqué de la tension superficielle de l'eau. Il est bien connu que dans une composition de liquide émulseur destiné à la production de mousse extinctrice, la résistance du film aqueux formé à la surface des hydrocarbures ne dépend pas uniquement du pouvoir du tensioactif d'abaisser la tension superficielle de la mousse extinctrice, mais aussi de l'influence des cotensioactifs utilisés. Nous avons constaté que la formule moléculaire d'un tensioactif peut avoir un effet remarquable dans ce domaine, ce qui est l'objet de la présente invention.

Le brevet FR2.866.883 des mêmes auteurs, décrit des molécules présentant une partie hydrophile monomère. Nous avons préparé des molécules présentant une partie hydrophile de structure analogue, mais polymérisée (exemples 1 à 9) et nous avons comparé les résultats selon une procédure de contrôle décrite dans l'exemple 10 comme une méthode de mesure de la résistance d'un film aqueux à la surface d'un hydrocarbure. Les résultats montrent que le film obtenu avec le tensioactif polymérisé est plus résistant que celui obtenu avec le tensioactif monomère, la mesure de la tension superficielle de l'eau obtenue avec le dernier étant plus basse que celle obtenue avec le premier.

Les tensioactifs perfluoroalkylés de la présente invention sont préparés :
- soit à partir d'époxydes perfluoroalkylés tels que : 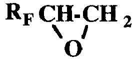 (FR 2.529.890), 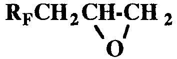 (US 3.388.078), 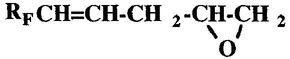 (US 4.038.195), 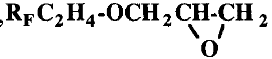 (Ger.Offen. 2.405.042), 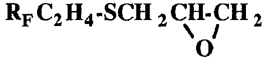 (US 3.906.049), 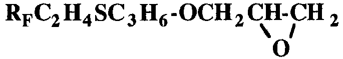 (US 4.577.036), 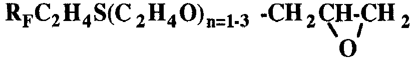 (F. Szönyi et Coll., Tenside Surf. Deter. 31 (1994) 2 p.124).
- soit à partir d'époxydes possédant une double liaison terminale comme le groupement allyle du 1-allyloxy-2,3-époxypropane, ou deux doubles liaisons terminales comme le groupement diallylamine de la glycidyldiallylamine. Le cycle époxyde est ouvert par une amine primaire ou tertiaire contenue dans la partie polyamide hydrophile.

On utilise comme agent perfluoroalkylant les iodures de perfluoroalcane **CₙF₂ₙ₊₁I** qui sont des matières premières obtenues par le procédé de télomérisation du tétrafluoroéthylène illustré dans "Fluorocarbons and their Derivatives" par R.E.Banks, London, 1964, p.56-61. Les iodures de perfluoroalcane **CₙF₂ₙ₊₁I** dans lesquels **CₙF₂ₙ₊₁** est un groupement perfluoroalkyle linéaire ou ramifié où n est un nombre entier valant 4 à 20, ou un mélange de groupements perfluoroalkyles linéaires ou ramifiés, réagissent avec les doubles liaisons terminales soit en présence de radicaux libres de type azoïque ou peroxyde, selon des procédés bien connus (brevet US 3.145.222), soit par action simultanée de ces initiateurs de radicaux libres et de sel de dithionite (W.Y.Hung, J.Fluorine chem. 58 (1992) 1-8), de bisulfite (W.Y.Hung, J.Zhuang, Chin.J.Chem. 9(1991) 373), ou de dioxyde de thiourée (W.Y.Hung, J.Zhuang, Chin.J.Chem., 9(1991) 270).

Les tensioactifs amphotères de type carboxybétaïne correspondant à :
**A= X-E-CO-[NH-(CH₂)ₘ-N⁺(R₁)(R₂)-CH₂-CO]ₚ-NH-(CH₂)ₘ-N⁺(R₁)(R₂)-**
**B = -CH₂CH(OH)CH₂OCH₂CH(I)CH₂-, -CH₂CH(OH)CH₂OCH₂CH=CH₂-, -CH₂CH(OH)CH₂OC₂H₄**-, **-CH₂CH(OH)CH₂SC₂H₄-, -CH₂CH(OH)-, -CH₂CH(OH)CH₂(OC₂H₄)₁₋₃SC₂H₄-, -CH₂CH(OH)CH₂-, -CH₂CH(OH)CH₂CH=CH-,-CH₂CH(OH) CH₂-O- C₃H₆S C₂H₄-, -CH₂CH(OH) CH₂-N=**
**D = -CH₂CH(I)CH₂-** et/ou **--CH₂CH=CH-** lorsque **B** est un groupement **-CH₂CH(OH)CH₂-N=**, néant dans les autres cas;
**E** est un groupement -(CH₂)_{q} -, 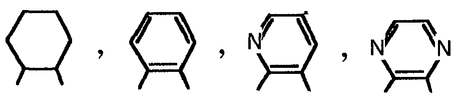
**X** = groupement alkyle ou aryle lorsque **E** est néant ou ⁻ **OOC** lorsque **E** est différent de néant.
sont préparés en trois étapes :
On fait d'abord réagir dans la N,N-diméthylformamide une diamine de formule

   **H₂N-(CH₂)ₘ-N(R₁)(R₂)**

   où m = 2 ou 3, R₁ et R₂ = alkyle mineur, avec un anhydride d'acide qui peut être l'anhydride succinique, glutarique, 1,2-cyclohexane dicarboxylique, phtalique, pyridinique, ou 2,3-pyrazine dicarboxylique.
Le composé qui précipite est le N,N-dialkylaminoamidoacide de formule

   **Y-X-E-CONH-(CH₂) ₘ-N(R₁)(R₂)** ***(I)***

   18 où **E** et **X** sont définis ci-dessus et **Y =** néant quand **E** est égal à néant et **H** quand **E** différent de néant.
Les composés **(*I*)** sont mis à réagir avec deux à trois équivalents d'un monochloroacétate d'alkyle pour donner un N,N-dialkylaminopolyamidoacide de formule

   **Y-X-E-CO[NH-(CH₂)ₘ-N⁺(R₁)(R₂) CH₂CO]ₚ-NH-(CH₂)ₘ-N(R₁)(R₂)** ***(II)***

   lequel est dissous dans le 2-propanol.
Afin de quaterniser le groupement amine tertiaire **-N(R₁)(R₂)** on ajoute lentement, soit le 1-allyloxy-2,3-époxypropane pour obtenir les composés de formule suivante :

   **Y-X-E-CO[NH-(CH₂)ₘ-N⁺(R₁)(R₂)CH₂CO]ₚ-NH-(CH₂)ₚ-N(R₁)(R₂)-CH₂CH(OH)CH₂O CH₂CH= CH₂** **(*III*),**

   soit le glycidyldiallylamine pour obtenir les composés de formule suivante :

   **Y-X-CO[NH-(CH₂)ₘ-N⁺(R₁)(R₂)CH₂CO]ₚ-NH-(CH₂)ₘ-N(R₁)(R₂)-CH₂CH(OH)CH₂N (CH₂CH= CH₂)₂** **(*IV*),**

   soit un époxyde perfluoroalkylé de formule suivante : 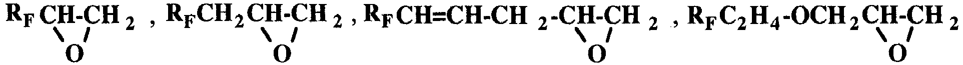 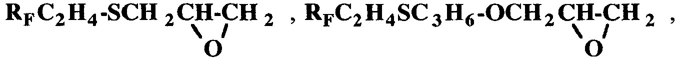 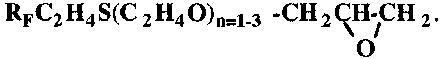
On obtient alors les tensioactifs perfluoroalkylés de type carboxybétaïne selon l'invention, avec la formule suivante :

   **A-B-(D-R_{F})ᵣ** ***(V)***

   avec **A, B, D, E** et **X** tel que défini précédemment, par perfluoroalkylation de la double liaison allylique du composé *(*III*)* ou *(IV)* par les iodures de perfluoroalkyle en présence de 2,2'-azobis(isobutyronitrile) et d'hydrosulfite de sodium, ou directement dans le cas de l'utilisation d'époxydes perfluoroalkylés.

Le mélange obtenu est dilué avec une petite quantité de monobutyléther du diéthylène glycol et complété avec de l'eau de telle sorte que la matière active des tensioactifs perfluoroalkylés de type carboxybétaïne de l'invention soit d'environ 40% avec une teneur en fluor d'environ 9%. Le pH du mélange est réglé entre 7 et 8.

Les tensioactifs amphotères de type carboxybétaïne correspondant à :
**A= M⁺⁻OOC-CH₂-[N⁺(R₁)(R₂)-(CH₂)ₘ- NHCOCH₂]_{P}N⁺(R₁)(R₂)-(CH₂)ₘ-NH-**
**B** = **-CH₂CH(OH)CH₂OC₂H₄** -, **-CH₂CH(OH)CH₂SC₂H₄-, -CH₂CH(OH)-, -CH₂CH(OH)CH₂(OC₂H₄)₁₋₃SC₂H₄-, -CH₂CH(OH)CH₂-, -CH₂CH(OH) CH₂CH=CH-, -CH₂CHOHCH₂-CH=CH-, -CH₂CH(OH) CH₂-O- C₃H₆S C₂H₄-, -CH₂CH(OH) CH₂-N=**
**D** = **-CH₂CH(I)CH₂-** et/ou --**CH₂CH**=**CH-** lorsque **B** est un groupement **-CH₂CH(OH) CH₂-N=,** néant dans les autres cas,
**M**⁺ **= un ion alcalin ou néant**
sont obtenus en trois étapes :
on fait réagir la glycidyldiallylamine ou les époxydes de formules suivantes : 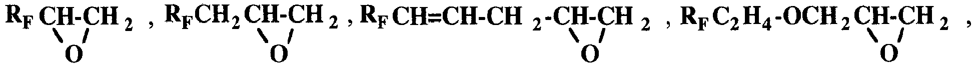 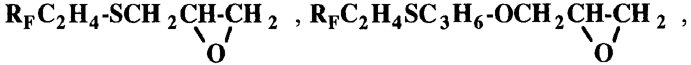 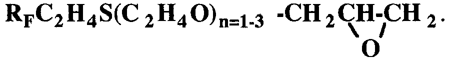 avec plusieurs équivalents d'une diamine de formule

   **H₂N-(CH₂)ₘ-N(R₁)(R₂)**

   où m = 2 ou 3, R₁ et R₂ = alkyle mineur, pour obtenir l'intermédiaire de formule suivante

   **(R₁)(R₂)N⁺- (CH₂)ₘ-NH-B- (D)ᵣ** ***(VI)***

   qui est mis à réagir avec plusieurs équivalents d'un monochloroacétate d'alkyle en une ou plusieurs fois pour donner un N,N-dialkylaminopolyamidoacide de formule

   **⁻OOC-CH₂ [(R₁)(R₂)N⁺- (CH₂)ₘ-NHCOCH₂]ₚ- (R₁)(R₂)N⁺-(CH₂)ₘ-NH - B- (D)ᵣ** ***(VII)***
Les éventuelles double liaisons allyliques du composé ***(VII)*** sont ensuite perfluoroalkylées à l'aide des iodures de perfluoroalcane en présence de 2,2'azobis(isobutyronitrile) et de l'hydrosulfite de sodium. Enfin, en traitant le mélange à l'aide de l'hydroxyde de sodium ou de potassium, on obtient les tensioactifs perfluoroalkylés de type carboxylate de sodium ou de potassium selon l'invention. Le mélange est dilué avec une petite quantité de monobutyléther du diéthylène glycol et complété avec de l'eau de telle sorte que la matière première active des tensioactifs perfluoroalkylés carboxybétaïne de l'invention soit d'environ 40% avec une teneur en fluor d'environ 9%. Le pH du mélange est réglé entre 7 et 8.

On remarquera que les enchaînements **-CH₂CH(I)CH₂-** et **-CH=CH-CH₂-** éventuellement contenus dans **B et D** sont une description idéale, car selon le caractère acide ou basique du milieu réactionnel un pourcentage variable d'iode contenu dans la molécule pourra être éliminé pour conduire à un mélange de ces deux enchaînements.

Les exemples suivants illustrent les procédés de préparation des nouveaux tensioactifs perfluoroalkylés de la présente invention. Les parties et les pourcentages mentionnés sont en poids sauf indication contraire.

### Exemple 1

Préparation d'un tensioactif perfluoroalkylé de type carbobétaine correspondant à
**R_{F} = C₆F₁₃**
**A = ⁻OOC-E-CO-[NH-(CH₂)₃-N⁺(CH₃)₂-CH₂-CO]₂-NH-(CH₂)₃-N⁺(CH₃)₂-**
**B** = - **CH₂CH(I)CH₂OCH₂CH(OH)CH₂ et/ou -CH=CH-CH₂OCH₂-CH(OH)CH₂-**
**D = néant**
E= 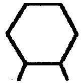
**r = 1**

Dans un réacteur muni d'un réfrigérant et d'un agitateur, on introduit 4,62 pp d'anhydride 1,2-cyclohexane dicarboxylique et 5 pp de diméthylformamide. On ajoute ensuite lentement 3,06 pp de N,N-diméthylpropylènediamine. La réaction est exothermique. La température s'élève jusqu'à 75°C. On maintient le mélange autour de 80°C pendant 3 heures. On ajoute au mélange 6,12 pp de N,N-diméthylpropylènediamine puis on ajoute goutte à goutte 7,35 pp de monochloroacétate d'éthyle en maintenant la température autour de 80°C. Le mélange est alors porté et maintenu à 100°C pendant 6 heures. Le milieu réactionnel est alors ramené à une température de 50°C et on rajoute 5 pp d'alcool isopropylique. On ajoute ensuite lentement 3,42 pp de 1-allyloxy-2,3-époxypropane. La réaction est légèrement exothermique. Le mélange est chauffé à 85°C pendant 3 heures. On laisse revenir la température à 60°C puis on ajoute successivement 12,15 pp d'iodure de perfluorohexane, 0,25 pp de 2,2'-azobis (isobutyronitrile) et 3,0 pp d'hydrosulfite de sodium dissous dans 9,5 pp d'eau et on chauffe ensuite entre 75 et 80°C pendant 3 heures. Lorsque le mélange est revenu à température ambiante, on ajoute 3,8 g d'hydroxyde de potassium à 90% dissous dans 11,35 g d'eau. On ajoute alors 3,9 pp de monobutyléther du diéthylène.

### Exemple 2

Préparation d'un tensioactif perfluoroalkylé de type carbobétaine correspondant à
**R_{F}** = **C₆F₁₃**
**A = ^{.}OOC-E-CO-[NH - (CH₂)₃-N⁺(CH₃)₂** - **CH₂**- **CO**]**₂**- **NH - (CH₂)₃-N⁺(CH₃)₂-**
**B = - CH₂CH(I)CH₂OCH₂CH(OH)CH₂ et/ou -CH=CH-CH₂OCH₂-CH(OH)CH₂-**
**D = néant**
**E=** 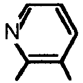
**r = 1**

Dans un réacteur muni d'un réfrigérant et d'un agitateur, on introduit 2,24 pp d'anhydride 2,3-pyridine dicarboxylique à 97% et 2,5 pp de diméthylformamide. On ajoute ensuite lentement 1,53 pp de N,N-diméthylpropylènediamine. La réaction est exothermique. La température s'élève jusqu'à 55°C. On maintient le mélange autour de 80°C pendant 3 heures. On ajoute au mélange 3,06 pp de N,N-diméthylpropylènediamine puis on ajoute goutte à goutte 3,68 pp de monochloroacétate d'éthyle en maintenant la température autour de 80°C. Le mélange est alors porté et maintenu à 100°C pendant 6 heures. Le milieu réactionnel est alors ramené à une température de 50°C et on rajoute 2,5 pp d'alcool isopropylique. On ajoute ensuite lentement 1,71 pp de 1-allyloxy-2,3-époxypropane. La réaction est légèrement exothermique. Le mélange est chauffé à 85°C pendant 3 heures. On laisse revenir la température à 60°C puis on ajoute successivement 6,07 pp d'iodure de perfluorohexane, 0,125 pp de 2,2'-azobis (isobutyronitrile) et 3,0 pp d'hydrosulfite de sodium dissous dans 9,5 pp d'eau et on chauffe ensuite entre 75 et 80°C pendant 3 heures. Lorsque le mélange est revenu à température ambiante, on ajoute 1,9 g d'hydroxyde de potassium à 90% dissous dans 5,6 g d'eau. On ajoute alors 4,75 pp de monobutyléther du diéthylène.

### Exemple 3

Préparation d'un tensioactif perfluoroalkylé de type carbobétaine correspondant à
**R_{F} = C₆F₁₃**
**A = ⁻OOC**-**E**-**CO**-[**NH**-(**CH₂**)**₃**-**N**⁺(**CH₃**)**₂**-**CH₂-CO**]**₂**-**NH-**(**CH₂**)**₃**-**N**⁺**(CH₃)₂**-
**B =-CH₂CH(I)CH₂OCH₂CH(OH)CH₂ et/ou** -**CH=CH**-**CH₂OCH₂**-**CH**(**OH**)**CH₂**-
**D = néant**
**E=** 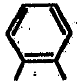
**r = 1**

Dans un réacteur muni d'un réfrigérant et d'un agitateur, on introduit 4,44 pp d'anhydride phtalique et 5 pp de diméthylformamide. On ajoute ensuite lentement 3,06 pp de N,N-diméthylpropylènediamine. La réaction est exothermique. La température s'élève jusqu'à 79°C. On maintient le mélange autour de 80°C pendant 1 heure. On ajoute au mélange 6,12 pp de N,N-diméthylpropylènediamine puis on ajoute goutte à goutte 7,35 pp de monochloroacétate d'éthyle en maintenant la température autour de 80°C. Le mélange est alors porté et maintenu à 100°C pendant 6 heures. Le milieu réactionnel est alors ramené à une température de 50°C et on rajoute 5 pp d'alcool isopropylique. On ajoute ensuite lentement 3,42 pp de 1-allyloxy-2,3-époxypropane. La réaction est légèrement exothermique. Le mélange est chauffé à 85°C pendant 3 heures. On laisse revenir la température à 60°C puis on ajoute successivement 12,15 pp d'iodure de perfluorohexane, 0,25 pp de 2,2'-azobis (isobutyronitrile) et 3,0 pp d'hydrosulfite de sodium dissous dans 9,5 pp d'eau et on chauffe ensuite entre 75 et 80°C pendant 3 heures. Lorsque le mélange est revenu à température ambiante, on ajoute 3,8 g d'hydroxyde de potassium à 90% dissous dans 11,35 g d'eau. On ajoute alors 4,05 pp de monobutyléther du diéthylène.

### Exemple 4

Préparation d'un tensioactif perfluoroalkylé de type carbobétaine correspondant à
**R_{F} = C₆F₁₃**
**A = ⁻OOC-E-CO-[NH-(CH₂)₃-N⁺(CH₃)₂-CH₂-CO]₂-NH-(CH₂)₃-N⁺(CH₃)₂-**
**B = - CH₂CH(I)CH₂OCH₂CH(OH)CH₂ et/ou -CH=CH-CH₂OCH₂-CH(OH)CH₂-**
**D = néant**
**E = -(CH₂)₃-**
**r = 1**

Dans un réacteur muni d'un réfrigérant et d'un agitateur, on introduit 3,42 pp d'anhydride glutarique et 5 pp de diméthylformamide. On ajoute ensuite lentement 3,06 pp de N,N-diméthylpropylènediamine. La réaction est exothermique. La température s'élève jusqu'à 78°C. On maintient le mélange autour de 80°C pendant 1 heure. On ajoute au mélange 6,12 pp de N,N-diméthylpropylènediamine puis on ajoute goutte à goutte 7,35 pp de monochloroacétate d'éthyle en maintenant la température autour de 80°C. Le mélange est alors porté et maintenu à 100°C pendant 6 heures: Le milieu réactionnel est alors ramené à une température de 50°C et on rajoute 5 pp d'alcool isopropylique. On ajoute ensuite lentement 3,42 pp de 1-allyloxy-2,3-époxypropane. La réaction est légèrement exothermique. Le mélange est chauffé à 85°C pendant 3 heures. On laisse revenir la température à 60°C puis on ajoute successivement 12,15 pp d'iodure de perfluorohexane, 0,25 pp de 2,2'-azobis (isobutyronitrile) et 3,0 pp d'hydrosulfite de sodium dissous dans 9,5 pp d'eau et on chauffe ensuite entre 75 et 80°C pendant 3 heures. Lorsque le mélange est revenu à température ambiante, on ajoute 3,8 g d'hydroxyde de potassium à 90% dissous dans 11,35 g d'eau. On ajoute alors 4,90 pp de monobutyléther du diéthylène.

### Exemple 5

Préparation d'un tensioactif perfluoroalkylé de type carbobétaine correspondant à
**R**_{F} **= 43% C₆F₁₃, 35% C₈F₁₇, 15% C₁₀F₂₁, 7% C₁₂F₂₅**
**A = ⁻OOC-E-CO-[NH-(CH₂)₃-N⁺(CH₃)₂**-**CH₂**-**CO**]**₂**-**NH**-(**CH₂)₃**-**N**⁺(**CH₃**)**₂**-
**B =- CH₂CH(I)CH₂OCH₂CH(OH)CH₂ et/ou -CH=CH-CH₂OCH₂-CH(OH)CH₂-**
**D = néant**
**E = -(CH₂)₂-**
**r = 1**

Dans un réacteur muni d'un réfrigérant et d'un agitateur, on introduit 3,00 pp d'anhydride succinique et 5 pp de diméthylformamide. On ajoute ensuite lentement 3,06 pp de N,N-diméthylpropylènediamine. La réaction est exothermique. La température s'élève jusqu'à 75°C. On maintient le mélange autour de 80°C pendant 3 heures. On ajoute au mélange 6,12 pp de N,N-diméthylpropylènediamine puis on ajoute goutte à goutte 7,35 pp de monochloroacétate d'éthyle en maintenant la température autour de 80°C. Le mélange est alors porté et maintenu à 100°C pendant 6 heures. Le milieu réactionnel est alors ramené à une température de 50°C et on rajoute 5 pp d'alcool isopropylique. On ajoute ensuite lentement 3,42 pp de 1-allyloxy-2,3-époxypropane. La réaction est légèrement exothermique, Le mélange est chauffé à 85°C pendant 3 heures. On laisse revenir la température à 60°C puis on ajoute successivement 14,44 pp un mélange d'iodures de perfluoroalcanes (43% C₆F₁₃I, 35% C₈F₁₇I, 15% C₁₀F₂₁I, 7% C₁₂F₂₅I), 0,25 pp de 2,2'-azobis (isobutyronitrile) et 3,0 pp d'hydrosulfite de sodium dissous dans 9,5 pp d'eau et on chauffe ensuite entre 75 et 80°C pendant 3 heures Lorsque le mélange est revenu à température ambiante, on ajoute 5,5 pp de monobutyléther du diéthylène et 15,5 pp d'eau.

### Exemple 6

Préparation d'un tensioactif perfluoroalkylé de type carbobétaine correspondant à
**R_{F} = C₄F₉**
**A = ⁻OOC-E-CO-[NH-(CH₂)₃-N⁺(CH₃)₂-CH₂-CO]₂-NH-(CH₂)₃-N⁺(CH₃)₂-**
**B = -CH₂CH(OH) CH₂-N=**
**D = -CH₂CH(I)CH₂- et/ou --CH₂CH=CH**
**E = -(CH₂)₂-**
**r=2**

Dans un réacteur muni d'un réfrigérant et d'un agitateur, on introduit 2,00 pp d'anhydride succimque et 3,33 pp de diméthylformamide. On ajoute ensuite lentement 2,04 pp de N,N-diméthylpropylènediamine. La réaction est exothermique La température s'élève jusqu'à 68°C On maintient le mélange autour de 80°C pendant 2 heures On ajoute au mélange 4,08 pp de N,N-diméthylpropylènediamine puis on ajoute goutte à goutte 4,66 pp de monochloroacétate d'éthyle en maintenant la température autour de 80°C Le mélange est alors porté et maintenu à 100°C pendant 6 heures. Le milieu réactionnel est alors ramené à une température de 50°C et on rajoute 3,33 pp d'alcool isopropylique. On ajoute ensuite lentement 2,98 pp de glycidyldiallylamine La réaction est légèrement exothermique Le mélange est chauffé à 85°C pendant 3 heures. On laisse revenir la température à 60°C puis on ajoute successivement 12,7 pp d'iodure de perfluorobutyle, 0,31 pp de 2,2'-azobis (isobutyronitrile) et 3,66 pp d'hydrosulfite de sodium dissous dans 11,59 pp d'eau et on chauffe ensuite entre 75 et 80°C pendant 3 heures. Lorsque le mélange est revenu à température ambiante, on ajoute 5,5 pp de monobutyléther du diéthylène et 0,48 pp d'hydroxyde de potassium à 90% dissous dans 14,01 pp d'eau.

### Exemple 7

Préparation d'un tensioactif perfluoroalkylé de type carbobétaine correspondant à
**R_{F} = C₆F₁₃**
**A = ⁻OOC-E-CO-[NH-(CH₂)₃-N⁺(CH₃)₂**-**CH₂-CO]₂-NH-(CH₂)₃-N⁺(CH₃)₂-**
**B= - CH₂CH(I)CH₂OCH₂CH(OH)CH₂ et/ou -CH=CH-CH₂OCH₂-CH(OH)CH₂-**
**D = néant**
**E = -(CH₂)₂-**
**r = 1**

Dans un réacteur muni d'un réfrigérant et d'un agitateur, on introduit 3,00 pp d'anhydride succinique et 5 pp de diméthylformamide. On ajoute ensuite lentement 3,06 pp de N,N-diméthylpropylènediamine. La réaction est exothermique. La température s'élève jusqu'à 75°C. On maintient le mélange autour de 80°C pendant 3 heures. On ajoute au mélange 6,12 pp de N,N-diméthylpropylènediamine puis on ajoute goutte à goutte 7,35 pp de monochloroacétate d'éthyle en maintenant la température autour de 80°C. Le mélange est alors porté et maintenu à 100°C pendent 6 heures. Le milieu réactionnel est alors ramené à une température de 50°C et on rajoute 5 pp d'alcool isopropylique. On ajoute ensuite lentement 3,42 pp de 1-allyloxy-2,3-époxypropane. La réaction est légèrement exothermique. Le mélange est chauffé à 85°C pendant 3 heures. On laisse revenir la température à 60°C puis on ajoute successivement 12,15 pp d'iodure de perfluorohexane, 0,25 pp de 2,2'-azobis (isobutyronitrile) et 3,0 pp d'hydrosulfite de sodium dissous dans 9,5 pp d'eau et on chauffe ensuite entre 75 et 80°C pendant 3 heures. Le mélange est alors refroidi à 30-40°C, on ajoute 3,8 pp de potasse caustique à 90% dissous dans 8,68 pp d'eau. Le mélange est chauffé à 70-75°C pendant 4 heures. Lorsque le mélange est revenu à température ambiante, on ajoute 5,5 pp de monobutyléther du diéthylène glycol.

### Exemple 8

Préparation d'un tensioactif perfluoroalkylé de type carbobétaine correspondant à
**R_{F}** = **C₄F₉**
**A = ⁻OOC-CH₂-[N⁺(R₁)(R₂)-(CH₂)ₘ-NHCO-CH₂]ₚ-N⁺(R₁)(R₂)-(CH₂)ₘ-NH-**
**B = -CH₂CH(OH) CH₂-N=**
**D = -CH₂CH(I)CH₂-** et/ou **-CH₂CH=CH**
**r = 2**

Dans un réacteur muni d'un réfrigérant et d'un agitateur, on introduit 5,82 pp de diallylamine, puis on ajoute 5055 pp d'épichlorhydrine à 30°C. La réaction est exothermique. La température est maintenue autour de 40°C jusqu'à ce que la réaction soit complète (contrôle par chromatographie en phase gazeuse). On ajoute alors 18,36 pp de N,N-diméthylpropylènediamine, on porte la température à 80-85°C pendant 4 heures, et on ajoute 22,05 pp de monochloroacétate d'éthyle par portions de sorte que la température ne dépasse pas 100-110°C. Le mélange est chauffé à 90-5°C pendant 6 heures. On refroidit à 50°C et on ajoute successivement 0,93 pp de 2,2'-azobis (isobutyronitrile) et 9,4 pp d'hydrosulfite de sodium dissous dans 30 pp d'eau et 38,10 pp d'iodure de perfluorobutane. La réaction est exothermique et la température atteind 60-70°C. On maintient la température à 75-80°C pendant 3 heures. Le mélange est refroidi à 30-40°C, on ajoute 15 pp de potasse caustique à 90% dissous dans 27 pp d'eau. Le mélange est chauffé à 75-80°C pendant 4 heures puis est refroidi à 25-30°C; on ajoute alors 15,6 pp de monobutyléther du diéthylène glycol, puis on complète le mélange avec 22,19 pp d'eau.

### Exemple 9

Préparation d'un tensioactif perfluoroalkylé de type carbobétaine correspondant à
**R_{F} = C₆F₁₃**
**A = ⁻OOC-E-CO-[NH - (CH₂)₃-N⁺(CH₃)₂ - CH₂- CO]₂- NH - (CH₂)₃N⁺(CH₃)₂-**
**B = - CH₂CH(I)CH₂OCH₂CH(OH)CH₂ et/ou -CH=CH-CH₂-OCH₂-CH(OH)CH₂-**
**D = néant**
**E = -(CH₂)₂-**
**r = 1**

Dans un réacteur muni d'un réfrigérant et d'un agitateur, on introduit 12,00 pp d'anhydride succinique et 20 pp de diméthylformamide. On ajoute ensuite lentement 12,24 pp de N,N-diméthylpropylènediamine. La réaction est exothermique. La température s'élève jusqu'à 80°C. On maintient le mélange autour de 80°C pendant 1 heure. On ajoute au mélange 36,70 pp de N,N-diméthylpropylènediamine puis on ajoute goutte à goutte 44,10 pp de monochloroacétate d'éthyle en maintenant la température autour de 80°C. Le mélange est alors porté et maintenu à 100°C pendant 6 heures. Le milieu, réactionnel est alors ramené à une température de 60°C et on rajoute 20 pp d'alcool isopropylique. On ajoute ensuite lentement 13,68 pp de 1-alkyloxy-2,3-époxypropane. La réaction est légèrement exothermique. Le mélange est chauffé à 85-90°C pendant 3 heures. On laisse revenir la température à 60°C puis on ajoute successivement 48,60 pp d'iodure de perfluorohexane, 1,00 pp de 2,2'-azobis (isobutyronitrile) et 12,0 pp d'hydrosulfite de sodium dissous dans 38,00 pp d'eau ; la réaction est exothermique et la température s'élève à 70-80°C. On maintient ensuite la température entre 75 et 80°C pendant 3 heures. Le mélange est alors refroidi à 25-30°C, on ajoute 22,00 pp de monobutyléther du diéthylène, glycol et on complète le mélange avec 29,66 pp d'eau.

### Exemple 10

Préparation d'un tensioactif perfluoroalkylé de type cationique correspondant à
**R_{F} = C₆F₁₃**
**A = CH₃-CO-[NH- (CH₂)₃-N⁺(CH₃)₂ - CH₂- CO]₂- NH - (CH₂)₃-N⁺(CH₃)₂-**
**B = - CH₂CH(I)CH₂OCH₂CH(OH)CH₂ et/ou -CH=CH-CH₂OCH₂-CH(OH)CH₂-**
**D = néant**
**E = néant**

Dans un réacteur muni d'un réfrigérant et d'un agitateur, on introduit 3,06 pp d'anhydride acétique. On ajoute ensuite lentement 3,06 pp de N,N-diméthylpropylènediamine. La réaction est exothermique. La température s'élève jusque 75°C. On maintient le mélange autour de 80°C pendant 3 heures. On ajoute au mélange 6,12 pp de N,N-diméthylpropylènediamine puis on ajoute goutte à goutte 7,35 pp de monochloroacétate d'éthyle en maintenant la température autour de 80°C. Le mélange est alors porté et maintenu à 100°C pendant 6 heures. Le milieu réactionnel est alors ramené à une température de 50°C et on rajoute 5 pp d'alcool isopropylique. On ajoute ensuite lentement 3,42 pp de 1-allyloxy-2,3-époxypropane. La réaction est légèrement exothermique. Le mélange est chauffé à 85°C pendant 3 heures. On laisse revenir la température à 50°C puis on ajoute successivement 12,15 pp d'iodure de perfluorohexane, 0,25 pp de 2,2'-azobis (isobutyronitrile) et 3,0 pp d'hydrosulfite de sodium dissous dans 9,5 pp d'eau et on chauffe ensuite entre 75 et 80°C pendant 3 heures. Lorsque le mélange est revenu à température ambiante, on ajoute 3,8 g d'hydroxyde de potassium à 90% dissous dans 16,29 g d'eau. On ajoute alors 5,5 pp de monobutyléther du diéthylène.

### Exemple 11

Description et résultats d'une méthode de mesure de la résistance d'un film aqueux à la surface d'un hydrocarbure

On prépare la solution à tester, ici un émulseur AFFF 3%, en mélangeant les composés suivants 6,0 pp d'un tensioactif fluoré, 12,0 pp d'un tensioactif anionique^{a)}, 0,8 pp d'un tensioactif de type amphotère^{b)}, 30,0 pp de monobutyléther du diéthylène glycol et 51,2 p d'eau. Le pH est réglé entre 7 et 8 On verse 50 ml de cyclohexane dans une boîte de Petri et on pose une vis de 2,5 mm de long au centre de la boîte, pointe en l'air. On fait ensuite tomber goutte à goutte 3 ml de la solution à tester sur le pointe de la vis, à l'aide d'une éprouvette Dès la première goutte on déclenche le chronomètre le temps utile à l'addition des 3 ml de solution est de 1 à 2 minutes. On éloigne la boîte de Petri de l'éprouvette, on enlève la vis avec précaution et on attend 1 minute. On approche une allumette enflammée à 10 mm de la surface du liquide, puis on balaie la surface : si le cyclohexane ne s'enflamme pas, on touche la surface du cyclohexane avec une allumette enflammée au bord de la boîte de Petri, tout en déclenchant le chronomètre On note le temps nécessaire pour allumer le liquide, soit x secondes, et on retire l'allumette. On observe alors l'avancement du front de flamme, lorsque la totalité de la surface a pris feu, on arrête le chronomètre et on note le temps y On utilise le couple x/y pour comparer la résistance au feu du film produit par différentes solutions sur le cyclohexane.

Le tableau suivant regroupe les résultats obtenus pour l'émulseur AFFF 3% formulé avec les tensioactifs fluorés polymérisés selon l'invention correspondant aux exemples 7 et 9, leur homologue monomère le FLUOTAN AM 600 (FR2 866 883) et un tensioactif polymérisé de structure différente, le FLUOTAN A825 (EP 0 390 905):

| Tensioactif fluoré utilisé dans la formulation | FLUOTAN A825 | FLUOTAN AM600 | Ex.7 | Ex.9 |
|---|---|---|---|---|
| Tension superficielle (solution 3%) en mN/m | 16,4 | 17,8 | 18,3 | 18,6 |
| Tension interfaciale (solution 3% cyclohexane) | 0,9 | 0,6 | 0,5 | 0,6 |
| Coefficient d'étalement | 7,7 | 6,6 | 6,2 | 5,8 |
| pH de la formulation | 7,5 | 7,5 | 7,5 | 7,5 |
| Foisonnement (solution 3%) | 8,5 | 9,5 | 9,0 | 7,5 |
| Temps de décantation 25% | 7'00" | 7'15" | 6'00" | 6'00" |
| Résiste au balayage de la flamme | oui | oui | oui | oui |
| Couple x/y en secondes | 14/28 | 13/28 | 25/49 | 7/23 |

| | | | | |
|---|---|---|---|---|
| ^{a)} sodium octyl sulfate (SULFETAL 4069 /Zschimmer & Schwartz) ^{b)} Coeoamidopropylbétaïne (AMONYL 380BA / Seppic) | | | | |

Dans le tableau ci-dessus, on peut voir de façon tout à fait inattendue que le tensioactif polymérisé de l'exemple 7 confère au film aqueux une plus grande résistance que celle induite par l'usage du FLUOTAN AM 600, analogue monomère du tensioactif de l'exemple 7, en dépit d'une tension superficielle plus élevée. Cette tendance se vérifie si l'on compare les résultats obtenus avec le FLUOTAN A825, et les exemples 7 et 9: les tensioactifs polymérisés dès exemples 7 et 9 induisent respectivement une résistance du film aqueux meilleure ou à peu près équivalente à celle obtenue avec le FLUOTAN A825, tensioactif polymérisé de structure différente, en dépit là aussi d'une tension superficielle beaucoup plus élevée. Ceci montre que la structure moléculaire des composés selon l'invention a un effet remarquable et inattendu sur la résistance du film aqueux.

## Revendications

1. Nouveaux tensioactifs perfluoroalkylés répondant à la formule générale suivante :
**A -B -(D- R_{F})ᵣ,**
**caractérisés par le fait qu'**ils sont hydrophobes et oléophobes, qu'ils contiennent une partie hydrophile polymérique,
où
**R_{F}** = chaîne perfluoroalkyle linéaire ou ramifiée **CₙF₂ₙ₊₁** où **n = 4 à 20,** ou un mélange de groupements perfluoroalkyle linéaires ou ramifiés ;
**A = X-E-CO-[NH - (CH₂)ₘ-N⁺(R₁)(R₂) - CH₂- CO]_{P}- NH - (CH₂)ₘ-N⁺(R₁)(R₂) -** ou **M^{+ -}OOC-CH₂-[N⁺(R₁)(R₂) - (CH₂)ₘ- NH CO - CH₂]_{P}- N⁺(R₁)(R₂) - (CH₂)ₘ₋ NH-**
**B = -CH₂CH(OH) CH₂OCH₂CH(I)CH₂-,**
**-CH₂CH(OH)CH₂OCH₂CH=CH₂-,** quand
**A** = **⁻OOC-E-CO-[NH-(CH₂)ₘ-N⁺(R₁)(R₂) -CH₂- CO]_{P}-NH - (CH₂)ₘ-N⁺(R₁)(R₂) - ;**
**-CH₂CH(OH) CH₂OC₂H₄ -,**
**-CH₂CH(OH) CH₂(OC₂H₄)₁₋₃ SC₂H₄-**
**-CH₂CH(OH) CH₂SC₂H₄-,**
**-CH₂CH(OH)-,**
**-CH₂CH(OH) CH₂-,**
**-CH₂CH(OH) CH₂CH=CH-,**
**-CH₂CH(OH) CH₂-O-C₃H₆S C₂H₄-,**
**-CH₂CH(OH) CH₂-N=** dans tous les cas ;
**D = -CH₂CH(I)CH₂-** et/ou **--CH₂CH=CH-** lorsque **B** est un groupement**-CH₂CH(OH) CH₂-N=,** néant dans les autres cas;
**E** = néant, **-(CH₂)_{q}** -, 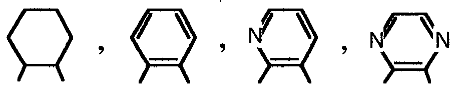
**M⁺ = un ion alcalin** ou **néant;**
**m, p** et **q** sont des nombres entiers valant 2 ou 3 indépendemment les uns des autres;
**r** est égal à 2 lorsque **B** est égal à **-CH₂CH(OH) CH₂-N=,** 1 dans les autres cas ;
**R₁** et **R₂** sont des groupements alkyle mineur identiques ;
**X =** groupement alkyle ou aryle lorsque **E** est néant ou **⁻OOC** lorsque **E** est différent de néant.

2. Procédé d'obtention de nouveaux tensioactifs perfluoroalkylés selon la revendication (1) **caractérisé par le fait que** le cycle époxydique du 1-allyloxy-2,3-époxypropane, de la glycidyldiallylamine ou des époxydes perfluoroalkylés de formules suivantes 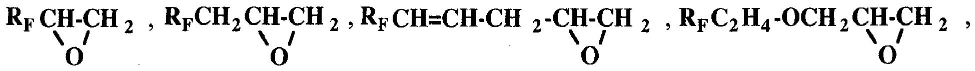 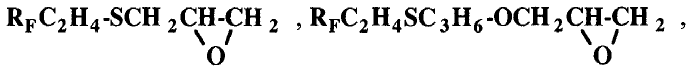 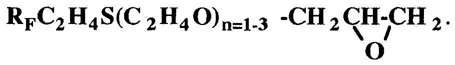 est formellement ouvert par une fonction amine primaire ou tertiaire contenue dans la partie hydrophile polymérique.

3. Procédé d'obtention de nouveaux tensioactifs perfluoroalkylés selon les revendications (1), et (2) **caractérisé par le fait que** la. N,N-dialkylalkylènediamine utilisée est la N,N-diméthyléthylènediamine, la N,N-diéthyléthylène diamine, la N,N-diméthylpropylènediamine ou la N,N-diéthylpropylènediamine.

4. Procédé d'obtention de nouveaux tensioactifs perfluoroalkylés selon les revendications (1),(2) et (3), **caractérisé par le fait que** le polyamide substitué est obtenu par réaction d'une N,N-dialkylalkylènediamine avec un anhydride d'acide suivie d'une polymérisation effectuée en une ou plusieurs fois par un monohalogénoacétate d'alkyle, puis quaternisation de la fonction amine tertiaire restant par un époxyde comportant une ou deux double liaisons terminales capables de réagir avec un iodure de perfluoroalcane ou un mélange d'iodures de perfluoroalcanes, ou par un époxyde perfluoroalkylé.

5. Procédé d'obtention de nouveaux tensioactifs perfluoroalkylés selon les revendications (1),(2) et (3), **caractérisé par le fait que** le polyamide substitué est obtenu par réaction d'une N,N-dialkylalkylènediamine avec un époxyde tel que la glycidyldiallylamine comportant deux double liaisons terminales capables de réagir avec un iodure de perfluoroalcane ou un mélange d'iodures de perfluoroalcanes, ou des époxydes perfluoroalkylés de formules suivantes : 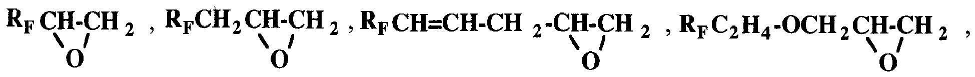 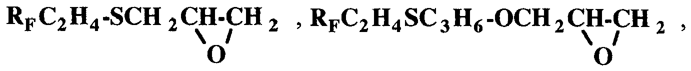 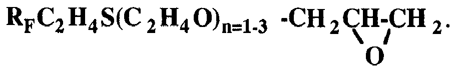 suivie d'une polymérisation effectuée en une ou plusieurs fois par un monohalogénoacétate d'alkyle, puis une hydrolyse avec un hydroxyde alcalin.

6. Procédé d'obtention de nouveaux tensioactifs perfluoroalkylés selon les revendications (1), (2), (3) et (4) **caractérisé par le fait que** les anhydrides d'acide sont ceux des acides acétique, benzoïque butyrique, caprique, caproïque, heptanoïque, isobutyrique, isovalérique, propionique, valérianique, succinique, glutarique, maléique, phtalique, pyridinique, 1,2-cyclohexane dicarboxylique, 2,3-pyrazine dicarboxylique.

7. Procédé d'obtention de nouveaux tensioactifs perfluoroalkylés selon les revendications (1), (2), (3), (4) et (5), **caractérisés par le fait que** la ou les doubles liaisons allyliques terminales présentes dans les intermédiaires de l'invention est ou sont soumises à une réaction d'addition avec un iodure de perfluoroalcane ayant 4 à 20 atomes de carbone ou un mélange d'iodures de perfluoroalcanes.

8. Utilisation des tensioactifs perfluoroalkylés selon la revendication (1) comme agents hydrophobes et oléophobes ou comme additifs dans les mousses extinctrices pour améliorer leur résistance anti-incendie.

## Patentansprüche

1. Neue perfluoralkylisierte Tenside, die der folgenden allgemeinen Formel entsprechen:
**A -B -(D- R_{F})ᵣ,**
und **dadurch gekennzeichnet, dass** sie hydrophobe und oleophobe sind, dass sie einen hydrophilen und polymeren Bestandteil enthalten,
wo dabei
**R_{F}** = perfluoralkylisierte gerade oder verzweigte Kette **CₙF₂ₙ₊₁** wo **n** = **4** bis **20,** oder ein Gemisch aus geraden oder verzweigten perfluoralkylisierten Gruppen;
**A = X-E-CO-[NH - (CH₂)ₘ-N⁺(R₁)(R₂) - CH₂- CO]_{P}- NH - (CH₂)ₘ-N⁺(R₁)(R₂) - oder M^{+ -}OOC-CH₂-[N⁺(R₁)(R₂) - (CH₂)ₘ- NHCO - CH₂]_{P}- N⁺(R₁)(R₂) - (CH₂)ₘ- NH-**
**B = -CH₂CH(OH) CH₂OCH₂CH(I)CH₂-,**
**-CH₂CH(OH)CH₂OCH₂CH=CH₂-,** wo
**A = ⁻OOC-E-CO-[NH-(CH₂)ₘ-N⁺(R₁)(R₂) -CH₂- CO]_{P}-NH - (CH₂)ₘ-N⁺(R₁)(R₂) -;**
**-CH₂CH(OH) CH₂OC₂H₄ -,**
**-CH₂CH(OH) CH₂(OC₂H₄)₁₋₃ SC₂H₄-**
**-CH₂CH(OH) CH₂SC₂H₄-,**
**-CH₂CH(OH)-,**
**-CH₂CH(OH) CH₂-,**
**-CH₂CH(OH) CH₂CH=CH-,**
**-CH₂CH(OH) CH₂-O-C₃H₆S C₂H₄-,**
**-CH₂CH(OH) CH₂-N=** in allen Fällen;
**D** = **-CH₂CH(I)CH₂-** und/oder **--CH₂CH=CH-** wenn **B** eine Gruppe ist **-CH₂CH(OH)CH₂- N=** andernfalls nichts;
**E** = nichts, **-(CH₂)_{q}** -, 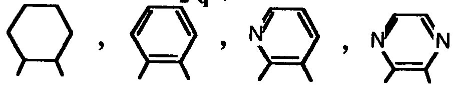
**M⁺ = ein alkali metal** oder **nichts;**
**m, p** und **q** sind ganze Zahlen mit dem Wert 2 oder 3 unabhängig voneinander;
**r** gleicht 2 wenn **B, -CH₂CH(OH) CH₂-N=** gleicht, andernfalls 1;
**R₁** und **R₂** sind kleinere gleiche Alkylgruppen;
**X** = Alkyl- bzw. Arylgruppe wenn **E** nichts ist oder **⁻OOC** wenn **E** anders als nichts ist.

2. Verfahren zur Herstellung von neuen perfluoralkylisierten Tensiden nach Anspruch (1), **dadurch gekennzeichnet, dass** der Epoxyring des 1-allyloxy-2,3-epoxypropans, des Glycidylallylamins oder der perfluooalkylisierten Epoxiden mit folgenden Formeln : 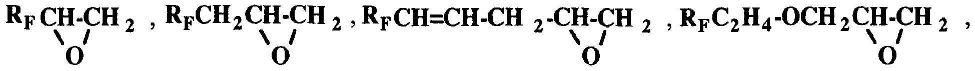 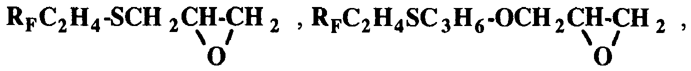 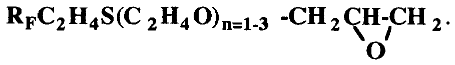 durch Umsetzung mit einer primären oder tertiären Aminogruppe, die in dem hydrophilen und polymeren Teil enthalten ist, geöffnet wird.

3. Verfahren zur Herstellung von neuen perfluoralkylisierten Tensiden nach den Ansprüchen (1) und (2), **dadurch gekennzeichnet, dass** das verwendete N,N-dialkylalkylenediamin das N,N-dimethylethylenediamin, das N,N-diethylendiamin, das N,N-dimethylpropylenediamin oder das N,N-diethylpropylenediamin ist.

4. Verfahren zur Herstellung von neuen perfluoralkylisierten Tensiden nach den Ansprüchen (1) bis (3), **dadurch gekennzeichnet, dass** das eingesetzte Polyamid durch das Umsetzen eines N,N-dialkylalkylenediamin mit eines Carbonsäureanhydrid erfolgt, bevor eine einmalige oder mehrmalige Polymerisation mit einer Alkyl Monohalogenoacetat durchgeführt wird und dann eine Quaterniesierung des tertiäres Amins mit einem Epoxyden, der aus einer oder zwei endständigen Doppelbindungen besteht, welche mit einem 1-Iodperfluoroalkan oder einem Gemisch von Iodperlluoroalkanen reagieren können.

5. Verfahren zur Herstellung von neuen perfluoralkylisierten Tensiden nach den Ansprüchen (1) bis (3), **dadurch gekennzeichnet, dass** das eingesetzte Polyamid durch Umsetzen eines N,N-dialkylalkylendiamin mit einem Epoxid in der Art der Glycidyldiallylamin, die aus zwei endständigen Doppelbindungen besteht, welche mit einem 1-Iodperfluoroalkan oder einem Gemisch von 1-Iodperfluoroalkanen reagieren können, oder mit perfluoralkylisierten Epoxiden der folgenden Formel: 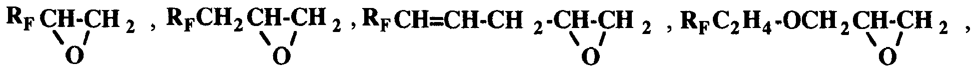 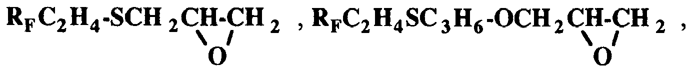 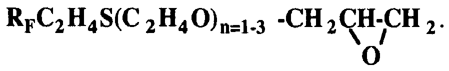 bevor eine einmalige oder mehrmalige Polymerisation mit einer Alkyl Monohalogenoacetat erfolgt und dann einer Hydrolyse mit einer Alkalimetalhydroxid.

6. Verfahren zur Herstellung von neuen perfluoralkylisierten Tensiden nach den Ansprüchen (1) bis (4), **dadurch gekennzeichnet, dass** die Carbonsäureanhydriden die von Essigsäure, Benzoesäure, Buttersäure, Caprinsäure, Capronsäure, Heptansäure, Isobuttersäure, 2-Methyl buttersäure, Propionsäure, Valeriansäure, Bernsteinsäure, Glutarsäure, Maleinsäure, Phtahalsäure, Pyridinsäure, 1,2-Cyclohexandicarbonsäure, 2,3-Pyrazindicarbonsäure sind.

7. Verfahren zur Herstellung von neuen perfluoralkylisierten Tensiden nach den Ansprüchen (1) bis (5), **dadurch gekennzeichnet, dass** die in den Produkten dieser Erfindung vorhandenen endständigen Doppelbindungen, einer Additionreaktion mit einer 1- Iodperfluoroalkan mit 4 bis 20 Kholenstoffatomen, oder mit einem Gemisch von 1-Iodperfluoroalkanen unterworfen sind.

8. Verwendung von neuen perfluoralkylisierten Tensiden nach Anspruch (1), als hydrophobe und oleophobe Leitungsmittel oder als Zusatzmittel bei der Herstellung von Feuerlöschschäumen zur Verbesserung ihrer Feuerbeständigkeit

## Claims

1. New perluoroalkyl surfactants corresponding to the following general formula:
**A -B -(D- R_{F})ᵣ,**
**characterised by** the fact they are hydrophobic and oleophobic, they contain an hydrophilic polymeric part,
where
**R_{F}** = linear or branched perfluoroalkyl chain **CₙF₂ₙ₊₁** where **n = 4 to 20,** or a mixture of linear or branched perfluoroalkyl groups;
**A = X-E-CO-[NH - (CH₂)ₘ-N⁺(R₁)(R₂) - CH₂- CO]_{P}- NH - (CH₂)ₘ-N⁺(R₁)(R₂) -** or **M^{+ -}OOC-CH₂-[N⁺(R₁)(R₂) - (CH₂)ₘ- NHCO - CH₂]_{P}- N⁺(R₁)(R₂)- (CH₂)ₘ- NH-**
**B = -CH₂CH(OH) CH₂OCH₂CH(I)CH₂-,**
**-CH₂CH(OH)CH₂OCH₂CH=CH₂-,** when
**A = ⁻OOC-E-CO-[NH-(CH₂)ₘ-N⁺(R₁)(R₂) -CH₂- CO]_{P}-NH - (CH₂)ₘ-N⁺(R₁)(R₂) - ;**
**-CH₂CH(OH) CH₂OC₂H₄ -,**
**-CH₂CH(OH) CH₂(OC₂H₄)₁₋₃ SC₂H₄-**
**-CH₂CH(OH) CH₂SC₂H₄-,**
**-CH₂CH(OH)-,**
**-CH₂CH(OH) CH₂-,**
**-CH₂CH(OH) CH₂CH=CH-,**
**-CH₂CH(OH) CH₂-O-C₃H₆S C₂H₄-,**
**-CH₂CH(OH) CH₂-N=** in all cases;
**D = -CH₂CH(I)CH₂-** and/or **--CH₂CH=CH-** when **B** is a group **-CH₂CH(OH)CH₂-N=,** none in other cases;
**E** = none, **-(CH₂)_{q}** -, 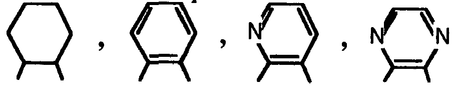
**M⁺ = an alkali metal or none;**
**m, p** et **q** are integers valued 2 or 3 separately from one another;
**r** is equal to 2 when **B** is equal to **-CH₂CH(OH) CH₂-N=,** 1 in other cases;
**R₁** et **R₂** are identical minor alkyl groups;
**X** = alkyl or aryl group when E is none or **⁻OOC** when E is different to none.

2. Process for obtaining new perfluoroalkyl surfactants according to the claim (1) **characterized by** the fact that the epoxide ring of 1-allyloxy-2,3-epoxypropane, glycidylallylamine or perfluoroalkyl epoxides with the following formulas 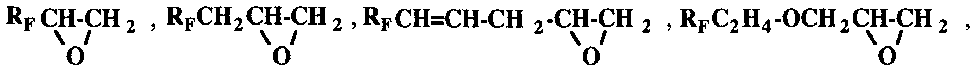 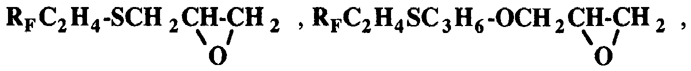 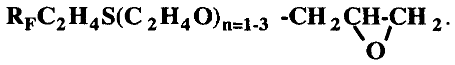 is formally opened by a primary or tertiary amine function contained in the hydrophilic polymeric part

3. Process for obtaining new perfluoroalkyl surfactants according to claims (1) and (2) **characterized by** the fact that the N,N-dialkylalkylènediamine used is N,N-dimethylethylenediamine, N,N-diethylethylenediamine, N,N-dimethylpropylenediamine or N,N-diethylpropylenediamine.

4. Process for obtaining new perfluoroalkyl surfactants according to claims (1), (2) and (3), **characterized by** the fact that the substituted polyamide is obtained by reacting a N,N-dialkylalkylenediamine with an acid anhydride followed by a polymerization reaction conducted in a single operation or in several operations with alkylmonohalogenoacetate, then a quaternisation reaction of remaining tertiary amine with an epoxide comprising one or two terminal double bonds, capable to react with a perfluoroalkyl iodide or a mixture of perfluoroalkyl iodides, or with a perfluoroalkyl epoxide.

5. Process for obtaining new perfluoroalkyl surfactants according to claims (1), (2) and (3), **characterized by** the fact that the substituted polyamide is obtained by reacting a N,N-dialkylalkylenediamine with an epoxide compound such as glycidyldiallylamine comprising two terminal double bonds capable to react with a perfluoroalkyl iodide or a mixture of perfluoroalkyl iodides, or perfluoroalkyl epoxide compounds with the following formula: 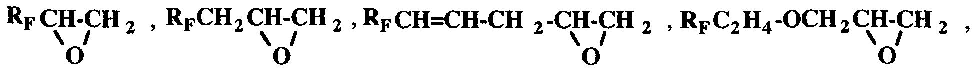 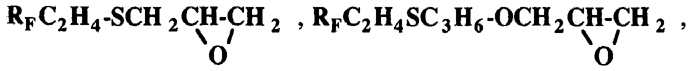 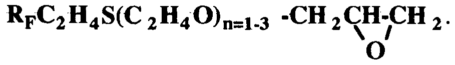 followed by a polymerization reaction conducted in a single operation or in several operations with an alkylmonohalogenoacetate, then hydrolysis with an alkali metal hydroxide.

6. Process for obtaining new perfluoroalkyl surfactants according to claims (1), (2), (3) and (4) **characterized by** the fact that acid anhydrides are from acetic, benzoic, butyric, capric, caproïc, heptanoic, isobutyric, isovaleric, propionic, valerianic, succinic, glutaric, maleic, phtalic, pyridinic, 1,2-cyclohexane dicarboxylic, 2,3-pyrazine dicarboxylic.

7. Process for obtaining new perfluoroalkyl surfactants according to claims (1), (2), (3), (4) and (5), **characterized by** the fact that the allylic terminal double bond(s) found in in the intermediates of the invention, is or are subjected to an addition reaction with a perfluoroalkyl iodide with from 4 to 20 carbon atoms or with a mixture of perfluoroalkyl iodides.

8. Use of perfluoroalkyl surfactants according to the claim (1) as hydrophobic and oleophobic agents or as additives in the fire-fighting foams to improve their fire resistance.
